# EUROPEAN PATENT APPLICATION

(11) **EP 1 512 366 A1**
(43) Date of publication of application: **09.03.2005**
(21) Application number: 04104272.2
(22) Date of filing: 03.09.2004
(51) Int. Cl.: A61B 1/04

(54) **Ophthalmic endoscope**

(30) Priority: 05.09.2003 IT BO20030521
(71) Applicant: KNIT VENTURES LLC, HOUSTON TEXAS 77002 (US)
(72) Inventor: Boscher, Claude, Via Salvemini 2-4, 60035 Jesi (IT)
(74) Representative: Jorio, Paolo, Dr. Ing.

(57) **Abstract**

An ophthalmic endoscope (10) having:
(a) an optical device for conveying a light beam, television images, and possibly a laser beam; and
(b) a handset (17), in turn having a cannula (22).

At the distal end (22a) of the cannula (22), the endoscope (10) has an optical assembly (23) to divert the light beam, television images, and possibly the laser beam.

## Description

The present invention relates to an ophthalmic endoscope.

As is known, an ophthalmic endoscope is used to examine the inside of the eye prior to surgery, and especially for determining the outcome of most surgical operations of the anterior and posterior chamber. It is also used after conventional surgery performed using a surgical microscope or under direct endoscopic control.

The major advantage of the endoscope according to the present invention is that it provides for viewing the more inaccessible anatomical areas of the eyeball (posterior of the iris, ciliary processes, and base of the vitreous humour) with no need for an angled cannula to follow the natural anatomical curvature of the eye. The present invention, in fact, employs a handset with a straight cannula, the tip of which is fitted with a number of lenses permitting 8-11 degree angle vision.

The endoscope according to the present invention provides for directly, and therefore accurately, controlling removal of the vitreous humour (vitrectomy); or, following liquid exchange and removal of the vitreous humour, for determining the presence of air bubbles possibly resulting in post-operative complications; and for dissecting and removing (peeling) retina membranes exerting pull between the retina and the vitreous humour.

Advantageously, though not necessarily, an accessory may be fitted to the cannula on the handset to provide a further surgical instrument on the endoscope, and may comprise, for example, a hook-shaped end for engaging and holding the tissue to be treated with the other hand.

Surgery is advantageously performed using a 532 nm wavelength laser which transmits energy along an optical fibre in the probe to photocoagulate portions of the retina with proliferations of neovessels. Such a laser also provides for cyclophotocoagulation treatment to strike and atrophy the ciliary bodies allowing aqueous humour into the posterior chamber of the eyeball, so as to reduce the pressure inside the posterior chamber in the treatment of glaucoma.

The endoscope according to the present invention has the further advantage of being sterilizable in an autoclave at 134°C for 18 minutes, as per regulations.

According to the present invention, there is provided an ophthalmic endoscope, as claimed in Claim 1.

A non-limiting embodiment of the present invention will be described by way of example with reference to the accompanying drawings, in which:
Figure 1 shows an overall view of the ophthalmic endoscope according to the present invention;
Figure 2 shows a larger-scale side view of a handset forming part of the Figure 1 endoscope;
Figure 3 shows a section of the Figure 2 handset;
Figure 4 shows a larger-scale portion of the Figure 2 handset;
Figure 5 shows a three-dimensional section of the Figure 4 portion;
Figure 6 shows a larger-scale side view of an optional accessory insertable inside the end portion of the Figure 2-5 handset;
Figure 7 shows a longitudinal section of the Figure 6 accessory.

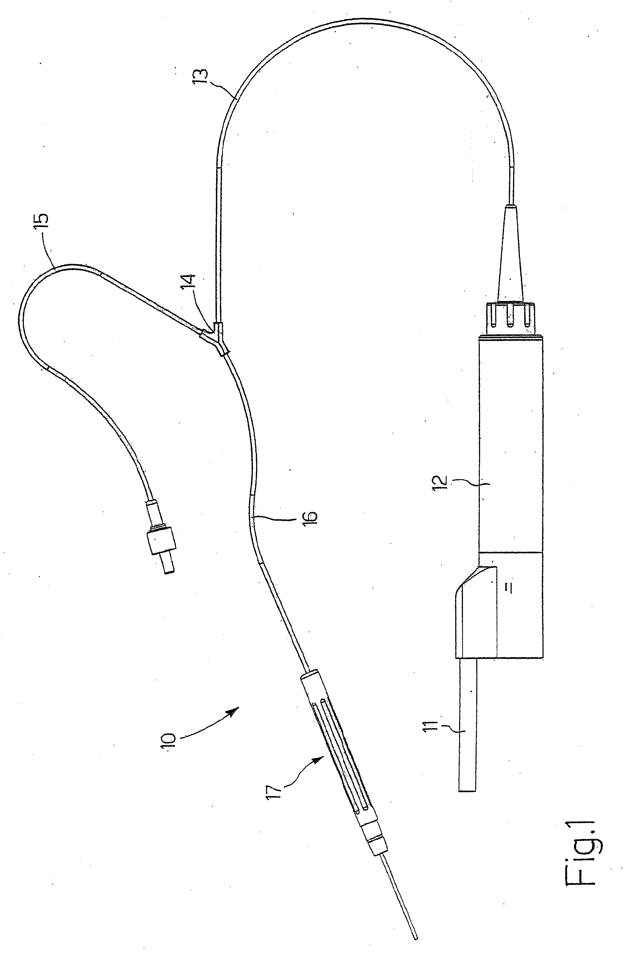

Number 10 in Figure 1 indicates an ophthalmic endoscope in accordance with the present invention.

Endoscope 10 comprises a conduit 11 containing light-carrying optical fibres (not shown), and which is connected to a television camera (not shown) for picking up images transmitted by further optical fibres (not shown).

Conduit 11 forms part of an optical device 12 comprising lenses (not shown) for eliminating reflection, and a lenticular image-release system (not shown).

At the opposite end to conduit 11, optical device 12 is connected to an optical fibre cable 13 for releasing light to illuminate the viewing field, and for picking up images by means of the television camera (not shown).

At a substantially Y-shaped connecting member 14, the optical fibres in cable 13 join the optical fibres contained in a cable 15 and for carrying a laser beam by which to operate on the eyeball.

Downstream from connecting member 14, the optical fibres in cables 13 and 15 continue together along a further cable 16 to a handset 17.

What is stated above is known and requires no further explanation.

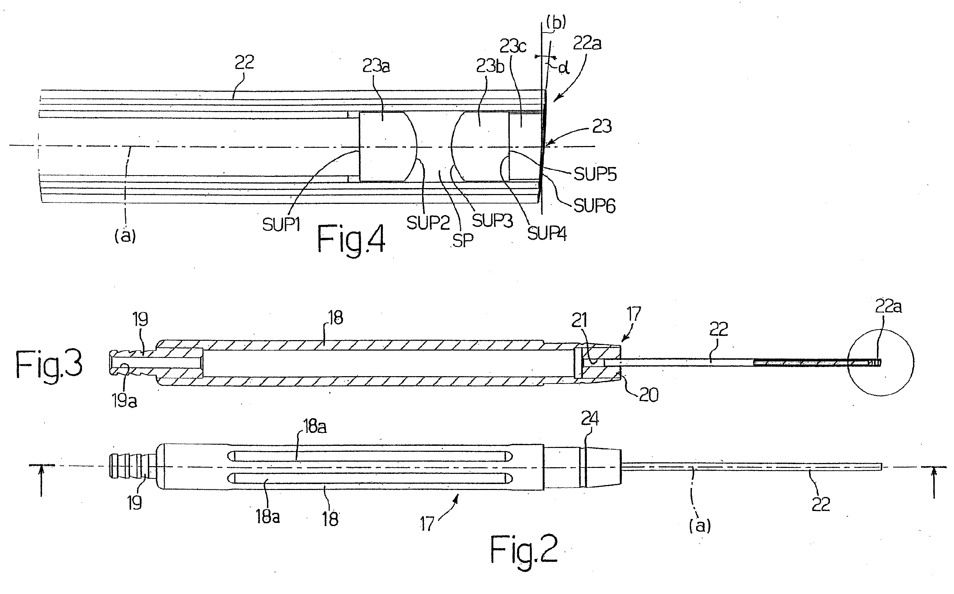

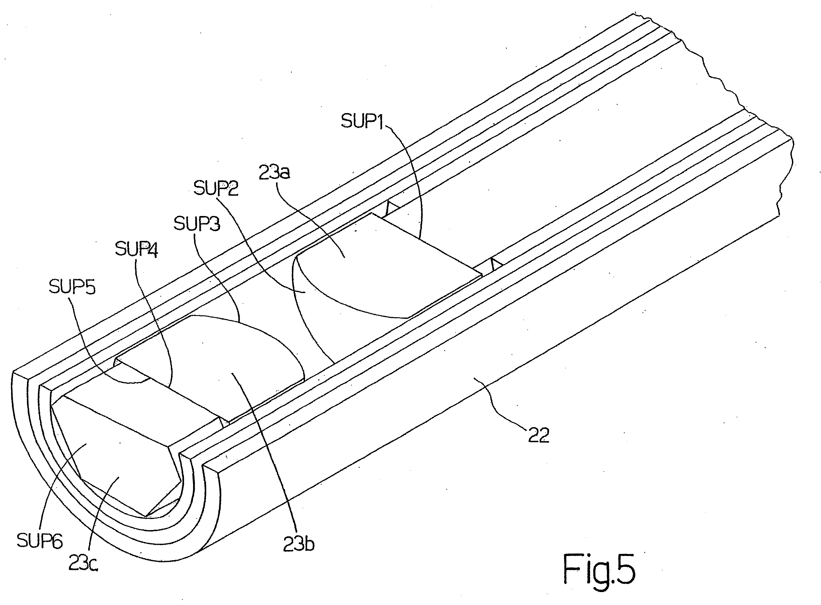

Handset 17 is shown in more detail in Figures 2 and 3.

More specifically, handset 17 comprises a substantially tubular main body 18 having a connecting member 19 for connection to cable 16; connecting member 19 comprises a longitudinal through hole 19a; and main body 18 is symmetrical with respect to a longitudinal axis (a) of symmetry.

As shown in Figure 2, the outer surface of main body 18 has a number of longitudinal grooves 18a to improve grip of handset 17 by the user (not shown).

At the opposite end to connecting member 19, main body 18 comprises a plug 20 having a through hole 21 into which a flexible cannula 22 is inserted.

Obviously, both the light and television image optical fibres, and the laser optical fibres extend through hole 19a, through hollow main body 18, and through cannula 22 to the distal end 22a of cannula 22.

The light, television image, and laser optical fibres are not shown in the accompanying drawings for the sake of simplicity.

As shown in Figures 4 and 5, the distal end 22a of flexible cannula 22 has an optical assembly 23 for diverting the light and laser beams by an angle of 8° to 11°.

Optical assembly 23 comprises a first lens 23a having a surface SUP1 on which the ends of the fibres (not shown) rest; and, on the opposite side to surface SUP1, first lens 23a has a surface SUP2 concave with respect to the incoming light beams.

Downstream with respect to longitudinal axis of symmetry (a), a second lens 23b has a surface SUP3 convex with respect to the incoming light beams; and an opposite surface SUP4 perpendicular to axis (a) and contacting a third lens 23c.

Lens 23c has a surface SUPS contacting surface SUP4 of lens 23b; and a surface SUP6 tilted at an angle α (of 5° in the example shown) with respect to an axis (b) perpendicular to axis (a).

By virtue of known optical laws, and because of the refraction coefficient - appropriately selected - of lens 23c and the tilt angle of surface SUP6, the light beams are obviously diverted by third lens 23c.

For example, by appropriately selecting the type of glass from which lens 23c is made, and with a tilt angle α of 5°, the beams are diverted roughly 10°.

As shown in Figure 2, main body 18 of handset 17 has a groove 24 substantially perpendicular to axis (a), for the purpose explained later on.

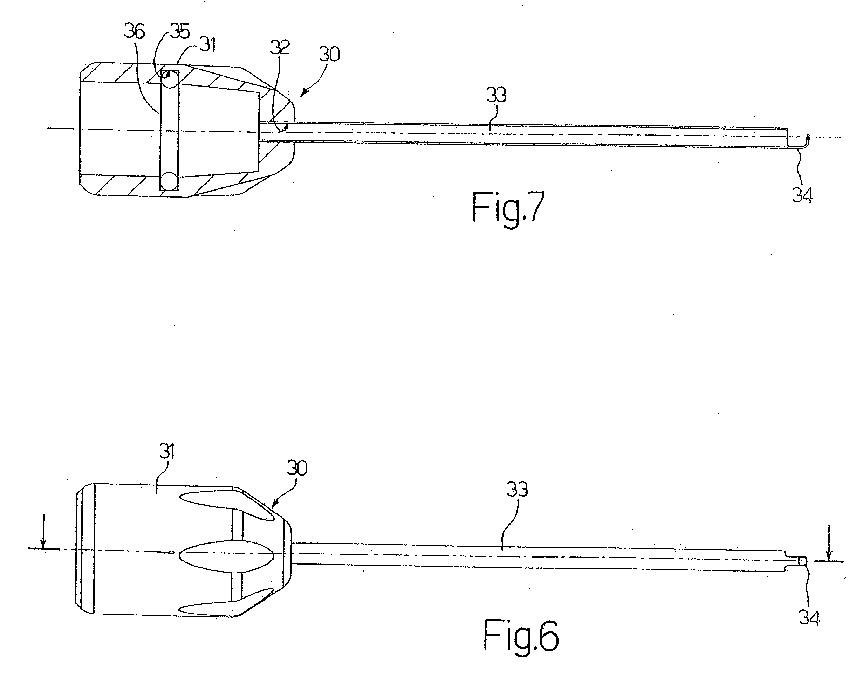

Figures 6 and 7 show a surgical operating accessory 30.

Accessory 30 comprises a cup 31 having a through hole 32 in the end; and a rigid rod 33 terminating with a hook 34 is inserted inside through hole 32.

Cup 31 also has an inner groove 35 housing an elastic O-ring.

Rod 33 is substantially the same length as flexible cannula 22.

Flexible cannula 22 can therefore be inserted inside cup 31 and rod 33, so that the end 22a of cannula 22 is located at hook 34.

O-ring 36 is force-fitted inside groove 24 to fix accessory 30 to handset 17.

Since the illumination light beam, images, and laser beam are diverted by optical assembly 23, the diverted laser beam (diverted by 8° to 11°) and hook 34 may be used either simultaneously or successively for surgical purposes.

## Claims

1. An ophthalmic endoscope (10) comprising:
(a) optical means for conveying a light beam, television images, and possibly a laser beam; and
(b) a handset (17) in turn comprising a cannula (22);
the endoscope (10) being **characterized in that** an optical assembly (23) is housed in the distal end (22a) of said cannula (22) to divert the light beam, television images, and possibly the laser beam.

2. An endoscope (10) as claimed in Claim 1, **characterized in that** the angle of diversion ranges between 8° and 11°.

3. An endoscope (10) as claimed in either of the foregoing Claims, **characterized by** also comprising a surgical accessory (30), in turn comprising a cup (31), and a rigid rod (33) terminating with a hook (34); said surgical accessory (30) being fitted onto said cannula (22).
